# EUROPEAN PATENT SPECIFICATION

(11) **EP 1 469 408 B1**
(45) Date of publication and mention of the grant of the patent: **27.03.2013**
(21) Application number: 04008743.9
(22) Date of filing: 13.04.2004
(51) Int. Cl.: G06Q 10/00

(54) **Pharmaceutical tracking system**
Pharmazeutisches Lokalisierungssystem
Système de suivi pharmaceutique

(30) Priority: 14.04.2003 US 463141 P
(43) Date of publication of application: 20.10.2004
(73) Proprietor: PatientSafe Solutions, Inc., California 92121 (US)
(72) Inventor: FORTH, Gerald E., Healdsburg, CA 95448 (US); SWENSON, David D., Encinitas, CA 92024 (US); STEUSLOFF, Patrick M., San Diego, CA 92128 (US)
(74) Representative: Cawley, Aimee Elizabeth

(56) References cited:
- US-A- 6 055 507
- US-A1- 2002 040 346
- US-A1- 2002 042 762
- US-A1- 2002 099 334

## Description

### Field of the Invention

The present invention relates generally to a product tracking system, and more particularly to a secure pharmaceutical tracking system.

### Description of the Related Art

The insertion of counterfeit drugs into the distribution chain between pharmaceutical manufacturers and patients has recently been spotlighted in the United States. This crime has many victims. Patients are treated with diluted, useless, and frequently hazardous tablets or solutions resulting in injury. Manufacturers are deprived of the revenues associated with the products only they are entitled to manufacture and sell. The health care providers must deal with the fear and uncertainty in their patients. This problem is estimated to cost billions of dollars per year. US-A-6,055,607 describes a system and method of dispensing, tracking and managing pharmaceutical product samples by communicatively linking prescribers and pharmacies to a central computing station.

Accordingly, what is needed is a system that securely tracks the distribution of pharmaceuticals from the manufacturer to the patient.

### Summary of the invention

In one aspect of the invention, there is provided a pharmaceutical product distribution system, comprising an activation computer configured to store authentication codes in a database and associate said authentication codes with pharmaceutical packages, wherein said activation computer is further configured to activate said authentication codes for pharmaceutical packages that are being sent to destination sites; and an authentication computer configured to receive authentication codes that are read from pharmaceutical packages received at said destination sites and determine whether said authentication codes have been activated. The system may comprise a system server including a first authentication module, a first code reader configured to read authentication codes from product packaging, an activation module configured to receive an authentication code read by the first code reader from product packaging prior to distribution and to transmit an activation request to the system server, the activation request comprising the authentication code, wherein the system server stores the authentication code as an active authentication code.

The system can also include a second code reader configured to read authentication codes from product packaging, and a second authentication module, configured to receive an authentication code read by the second code reader from product packaging and to receive active authentication codes from the system server. The second authentication module is configured to determine whether the authentication code read by the second code reader corresponds to the active authentication code received from the system server, and to notify a user of the second code reader as to whether the authentication code read by the second code reader corresponds to the active authentication code.

The second authentication module may be further configured to transmit an authentication request to the system server, wherein the authentication request can include an authentication code read by the second code reader. The first authentication module may also be further configured to determine whether the authentication code received with the authentication request corresponds to an active authentication code stored at the system server, wherein the first authentication module is configured to notify the second authentication module as to whether the authentication code from the second authentication module corresponds to the active authentication code.

In addition, the activation request can further include destination information, wherein the destination information is stored at the system server and transmitted to the second authentication module. The second authentication module Is further configured to determine whether information at the second code reader corresponds to the destination information received from the system server.

In another aspect of the invention, there is provided a method of tracking and authenticating pharmaceutical products from an origin location to a final destination, comprising: applying an authentication code to product packaging, reading the authentication code from the product packaging at the origin location prior to distribution, sending an activation request to a system server, wherein said activation request includes the read authentication code, and activating the read authentication code in response to the activation request, comprising storing the read authentication code as an active authentication code at the system server.

Another embodiment includes reading an authentication code from product packaging received at a receiving location, sending an authentication request to the system server, wherein the authentication request including the authentication code read from product packaging received at the receiving location, verifying the authentication request, by comparing the authentication code read from product packaging at the receiving location to the active authentication code, notifying the receiving location whether the authentication request was verified, by indicating whether the authentication code read from product packaging at the receiving location corresponds to the active authentication code, and expiring the active authentication code by storing the active authentication code at the system server as an expired authentication code.

In an additional embodiment of the method, the activation request further includes destination information, wherein the destination information is stored at the system server. The authorization request further includes receiving location information. In addition, verifying the authentication request can also include comparing the receiving location information to the destination information. In addition, notifying the receiving location can include indicating whether the receiving location information corresponds to the destination information stored at the server.

Another embodiment is a pharmaceutical product distribution system that includes: an activation computer having a module configured to receive an authentication code from a pharmaceutical package and to store the activation code to a system server as an active authentication code; and an authentication computer, having a module configured to receive the authentication code from the pharmaceutical package and communicate with the system server to determine if the pharmaceutical package is a counterfeit.

In one embodiment, the activation computer is configured to receive destination information for the pharmaceutical package along with the activation code.

### Brief Description of the Drawings

Figure 1 is a block diagram of one embodiment of a pharmaceutical tracking system.

Figures 2A-B are a flow diagram illustrating one method of operation of the pharmaceutical tracking system of Figure 1.

Figure 3 is a software flow diagram illustrating one embodiment of a method of processing an authentication code verification request at the server/database of the pharmaceutical tracking system.

### Detailed Description of the Preferred Embodiment

Embodiments of the invention will now be described with reference to the accompanying Figures, wherein like numerals refer to like elements throughout. The terminology used in the description presented herein is not intended to be interpreted in any limited or restrictive manner, simply because it is being utilized in conjunction with a detailed description of certain specific embodiments of the invention. Furthermore, embodiments of the invention may include several novel features, no single one of which is solely responsible for its desirable attributes or which is essential to practicing the inventions herein described.

Aspects of the invention include a secure system and method of supplying and tracking authentic pharmaceutical products from origin at a manufacturer to a provider such as a hospital, retail pharmacy, or nursing home. The system employs authentication codes, such as machine readable codes including bar codes, or radio frequency (RF) tags, which can be applied to any level of packaging of the products to be transported from the manufacturer, including single dosage packages. A system server/database issues a number of authentication codes to a manufacturer, and the manufacturer applies the codes to product packaging and/or containers for shipment. When the products are ready for shipment, the manufacturer activates the codes (or code, depending on how many are used) by reading the authentication code with a code reader, and transmitting the code along with additional information about the product such as expiration date, type of product or medication, and shipment information such as destination and time for shipment, for example, to the system server/database. Communication with the system server/database can take place over a secure web link, for example, to ensure the security of the authentication code being activated.

When the manufacturer ships the products directly to the provider, the provider can verify that the products received are those that were shipped by the manufacturer by reading the authentication codes on the shipping container, package, or single dose packages using a code reader. The code is transmitted to the system server/database, along with information regarding the provider location, and the system server/database provides verification as to whether the code read at the provider corresponds to the code activated by the manufacturer, and whether the provider location matches the destination location corresponding to the activated code.

Once the activated authentication code is read at the final destination specified by the manufacturer and stored in the server/database, the code is then expired in the system server/database. Substitute or counterfeit products can thus be identified because only the products received at the provider having the active authentication code will correspond to the authentication code activated by the manufacturer and stored in the system server/database. Attempts by a provider to authenticate a product having an expired authentication code will fail, thus notifying the provider that the product may be counterfeit.

When there are intermediate destinations in the distribution chain of a pharmaceutical product, authenticity can be verified at every location along the distribution chain. In addition, the location of the product can be tracked, as each time the activated authentication code is read by a code reader requesting verification from the system server/database, the physical location of the code reader that makes the request can be stored at the system server/database. Alternately, if a substitute or counterfeit authentication code is read by a code reader at an intermediate destination, the system server/database can indicate that the authentication code has not been activated by a manufacturer and is invalid. The server/database can notify the manufacturer and provider that a substitute product has attempted to enter the distribution chain.

Verification of an authentication code preferably includes correlation of at least one data element or informational element in addition to the authentication code, such as a product's intended destination. The data element or informational element is also preferably unrelated to or indiscernible from product packaging. Thereby, a would-be counterfeiter would only be able to copy one element (the authentication code) necessary for verification of a product from product packaging, and the additional data element would remain unknown. The additional data element or informational element is not limited to the intended destination or destinations of a product, and other types or categories of information are contemplated. In addition, the type or category of information used by the tracking system for verification of an authentication code may be altered periodically.

As used herein, an "input device" can be, for example, a keyboard, rollerball, mouse, voice recognition system, or other device capable of receiving information from a user and transmitting it to a computer. The input device can also be a touch screen associated with the display, in which case the user responds to prompts on the display by touching the screen. The user may enter textual information through the input device such as the keyboard or the touch-screen.

As used herein, "instructions" refer to computer-implemented steps for processing information in the system. Instructions can be implemented in software, firmware or hardware and include any type of programmed step undertaken by components of the system.

As used herein, a "microprocessor" may be any conventional general purpose single- or multi-chip microprocessor such as a Pentium® processor, a Pentium® Pro processor, a 8051 processor, a MIPS® processor, a Power PC® processor, or an ALPHA® processor. In addition, the microprocessor may be any conventional special purpose microprocessor such as a digital signal processor or a graphics processor. The microprocessor typically has conventional address lines, conventional data lines, and one or more conventional control lines.

As used herein, the term "module" refers to the various modules in the system as discussed in detail below. As can be appreciated by one of ordinary skill in the art, each of the modules comprises various sub-routines, procedures, definitional statements and macros. Each of the modules are typically separately compiled and linked into a single executable program. Therefore, the following description of each of the modules is used for convenience to describe the functionality of the preferred system. Thus, the processes that are undergone by each of the modules may be arbitrarily redistributed to one of the other modules, combined together in a single module, or made available in, for example, a shareable dynamic link library.

As used herein, the term "programming language" refers to any programming language such as C, C++, BASIC, Pascal, Java, and FORTRAN and ran under the well-known operating system. C, C++, BASIC, Pascal, Java, and FORTRAN are industry standard programming languages for which many commercial compilers can be used to create executable code.

As used herein, the term "code reader" refers to a device that reads machine readable codes. Preferably, the codes are affixed to a package or product. Examples of code readers include bar code readers, scanners, and two-dimensional code readers such as that described in U.S. Patent No. 6,601,772, issued on August 5, 2003.

### System Overview

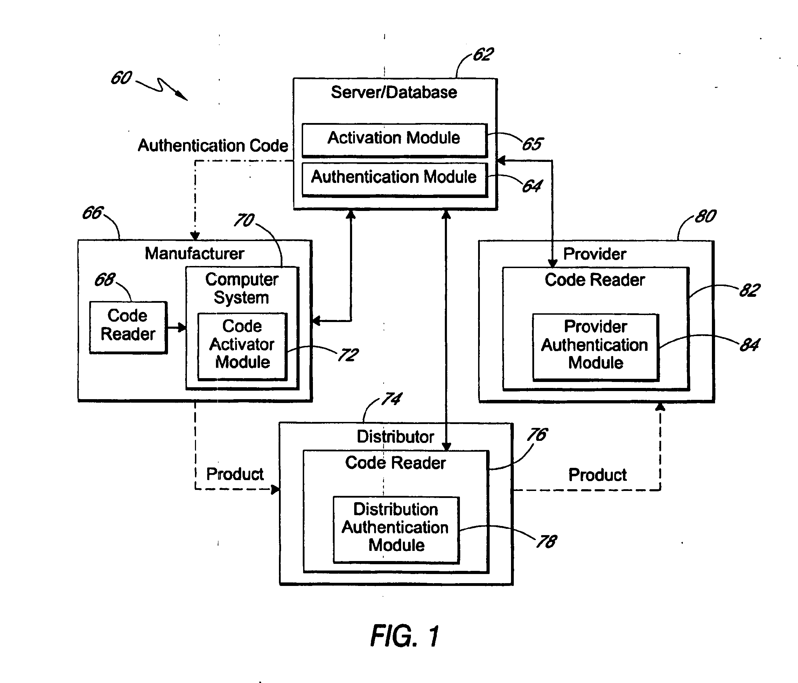

One embodiment of a secure pharmaceutical distribution and tracking system 60 is illustrated in Figure 1. The system 60 comprises a central system server/database 62 with an authentication module 64 and an activation module 65. The server/database 62 generates and provides a number of authentication codes to a manufacturer 66, wherein the authentication codes can be transmitted to the manufacturer electronically or distributed manually. The system 60 further comprises, at the manufacturer 66, a code reader 68 and a computer system 70, wherein the computer system 70 comprises a code activator module 72 configured to activate an authentication code in conjunction with the activation module 65 at the server/database 62.

The manufacturer 66 applies or affixes an authentication code to the product to be shipped or distributed, and inputs the affixed authentication code to the computer system 70 by reading the authentication code with the code reader 68. The code reader 68 is configured to communicate with the computer system 70 via a wireless link or wired connection, and may include an input device to receive information from a user, such as product information. The computer system 70 may also include a user input device configured to receive input from a user, such as user, product, manufacturer, and destination information. The computer system 70 and/or code reader 68 may also include memory for storing manufacturer information, wherein transmission of an authentication code from the code reader 68 or computer system 70 to the server/database 62 includes the stored manufacturer information.

The code activator module 72 receives an authentication code read by the code reader 68 at the manufacturer 66 and generates an activation request. The code activator module 72 transmits the activation request and authentication code, along with corresponding information such as type and quantity of the product, intended destinations, and time for shipment, to the system server/database 62. In one embodiment, the code activator module 72 modifies or encodes the activation request, authentication code, and corresponding information according to a predefined modification or encoding scheme prior to transmission to the server/database 62. The code activator module 72 also communicates with the system server/database 62 so as to receive confirmation that authentication codes are activated, notification of authentication code verification attempts by distributors and providers and the corresponding location of such requests, confirmation of products received at a provider, and expiration of authentication codes.

The activator module 72 may also be configured to query the system server/database 62 for information regarding the current location or tracking history for the shipped product and corresponding activated authentication codes, and may update additional information associated with an activated authentication code and stored at the server/database 62. The computer system 70 may also comprise a server for storing a list of activated authentication codes corresponding to individual products or dosages as part of a shipment, wherein the stored activated authentication codes can later be correlated with authentication codes affixed to products or dosages received at a distributor or final destination. Accordingly, the code activation module 72 can also be configured to store the activated authentication codes in the local server or database at the manufacturer 66.

The activation module 65 at the server/database 62 receives the activation request, authentication codes, and corresponding information from the code activator module 72 for activation of authentication codes in the tracking system 60. In response to receipt of the activation request, the activation module 65 flags the received authentication codes at the server/database 62 as "ACTIVE", and stores all or a portion of the additional information sent with the authentication code. The activation module 65 may be configured to decode the modification scheme used by the code activator module 72, when such a modification scheme is used, prior to storing the authentication codes and corresponding information.

The activation module 65 is also configured to communicate with the code activator module 72 so as to provide it with information, such as a list of confirmed ACTIVE authentication codes, confirmed delivery of the product at the provider, product end user information, and expiration of authentication codes.

The activation module 65 at the server/database 62 is further configured to transmit the ACTIVE authentication code and at least one additional piece of information, such as the intended final destination of the product associated with the authentication code, to designated verification entities in the shipping chain, such as distributors and a provider. For example, where a product is scheduled to be transferred from the manufacturer to a distributor, and from the distributor to the final destination, the server/database 62 transmits the ACTIVE authentication code and associated final destination information to the designated distributor and the final destination. Thereby, the authentication code affixed to the shipped product can be verified locally at the distributor and final destination upon receipt of the product rather than querying the database for verification of an authentication code. Due to the local storage of the authentication code, the time for completion of an authentication code verification and the overall data traffic to the database/server 62 are reduced. Furthermore, in the event the server/database 62 is unavailable, or the communication link between a code reader and the server/database 62 is inoperable, authentication codes can still be verified locally at the distributor and provider locations.

The tracking system 60 further comprises, at a distributor 74, a code reader 76 and a distribution authentication module 78, wherein the code reader 76 is configured to read the machine readable authentication codes affixed to products received from the manufacturer and to communicate with the server/database 62. In one embodiment, the distribution authentication module 78 is implemented at the code reader 76, and the code reader 76 is configured to store ACTIVE authentication modules and associated additional information as received from the server/database 62.

The code reader 76 is preferably configured to communicate with the server/database 62 via a combination of a wireless communication link and a network such as the Internet. In addition, the code reader 76 may include an input device configured to receive input from a user including, but not limited to, product, time and/or date of receipt, shipping, user, and current location information. The code reader 76 also preferably includes a real time clock and memory, and is configured to time stamp code reading events and store information about the distributor 74, such as location information.

The distribution authentication module 78 is configured to perform a verification procedure for an authentication code read by the code reader 76. In response to receiving an authentication code, the distribution authentication module 78 compares the received authentication code with ACTIVE authentication codes received from the server/database 62, and attempts to correlate any additional information, such as product type, distributor information, and final destination information with that received from the server/database 62. If the distribution authentication module 78 verifies that the authentication code read by the code reader 76 is ACTIVE and the additional information, such as quantity or destination information, is confirmed, the module 78 notifies the distributor of such verification and confirmation via either an audio or visual indicator at the code reader 76.

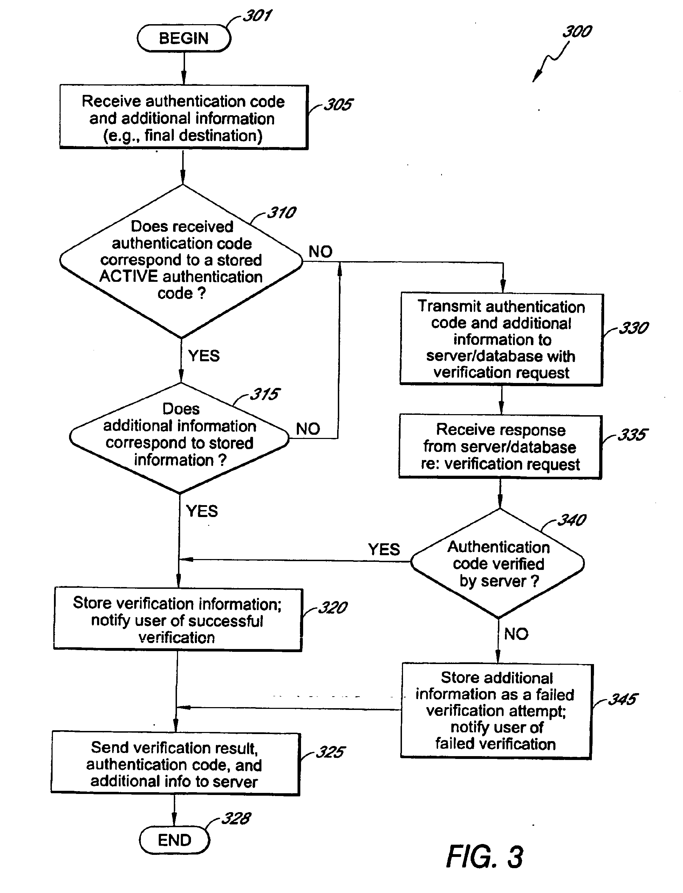

If the distribution authentication module 78 is unable to verify the authentication code, it queries the server/database 62 with the authentication code and additional information. In the event the server/database 62 verifies that the authentication code is ACTIVE and the additional information matches that stored at the server/database 62, the distribution authentication module 78 is notified and the code reader 76 in turn notifies the user that the authentication code has been verified. In the event the authentication code and/or additional information do not correlate with information at the server/database 62, the distribution authentication module 78 notifies the distributor that the authentication code is not ACTIVE, that the destination is incorrect, or that the quantity of units is incorrect. Thereby the distributor can identify substitute products or missing products and take the appropriate action. One embodiment of a method of verifying an authentication code is discussed in further detail hereinafter below in connection with Figure 3.

The tracking system 60 also comprises, at a provider 80, a code reader 82 having a provider authentication module 84, wherein the code reader 82 is configured to read the machine readable authentication codes affixed to products received, and to communicate with the server/database 62. The code reader 82 may include an input device configured to receive input from a user including, but not limited to, product, time and/or date of receipt, shipping, user, and current location information. The code reader 82 may also include a real time clock and be configured to time stamp code reading events, and may include memory for storing information about the provider 80, such as location information.

The provider authentication module 84 is configured to verify the authenticity of products received using the authentication code read by the code reader 82. The code reader 82 is preferably configured to communicate with the server/database 62 via a combination of a wireless communication link and a network such as the Internet.

Similar to the distribution authentication module 78, the provider authentication module 84 is configured to perform a verification operation for an authentication code read by the code reader 82. In response to receiving an authentication code, the provider authentication module 84 compares the received authentication code with ACTIVE authentication codes received from the server/database 62, and attempts to correlate any additional information, such as product type, provider information, and final destination information with that received from the server/database 62. If the provider authentication module 84 verifies that the authentication code read by the code reader 82 is ACTIVE and the additional information, such as quantity or destination information, is confirmed, the module 84 notifies the provider of such verification and confirmation via either an audio or visual indicator at the code reader 82.

If the provider authentication module 84 is unable to verify the authentication code, it queries the server/database 62 with the authentication code and additional information. In the event the server/database 62 verifies that the authentication code is ACTIVE and the additional information matches that stored at the server/database 62, the provider authentication module 84 is notified and the code reader 82 in turn notifies the user that the authentication code has been verified. In the event the authentication code and/or additional information do not correlate with information at the server/database 62, the provider authentication module 84 notifies the provider that the authentication code is not ACTIVE, that the destination is incorrect, or that the quantity of units is incorrect. Thereby the provider can identify substitute products or missing products and take the appropriate action. One embodiment of a method of verifying an authentication code is discussed in further detail hereinafter below in connection with Figure 3.

The provider authentication module 84 can also be configured to send end user information to the system server/database. Furthermore, the provider information will be the final destination information, and therefore entry of final destination information by the user may be unnecessary to verify an authentication code where the code reader 82 at the provider 80 stores provider information, and the authentication module 84 attempts to correlate the stored provider information with the final destination information received from the server/database. For example, where the tracking system 60 requires correlation of an authentication code with an ACTIVE authentication code, and correlation of final destination information with that provided by the manufacturer, then the user of the code reader at the provider need only read the authentication code with the code reader 82 in order to determine whether the authentication code is valid.

The authentication module 64 at the server/database 62 is configured to receive and process authentication requests from the distribution authentication module 78 and provider authentication module 84 in order to verify that an authentication code from such modules corresponds to an ACTIVE authentication code stored at the server/database 62. Verification of an authentication code includes determining whether the received authentication code is ACTIVE, and may also include correlation of product type, quantity, distributor or provider information, and final destination information with corresponding information stored at the server/database 62. The authentication module 64 may be configured to generate and send a notification message to the manufacturer in response to a verification request from a distributor or provider, wherein the message may include distributor or provider information in relation to a failed verification request, for example. In addition, the server/database 62 is configured to transmit information designated for the manufacturer 66 as received from distributors 66 and providers 80, such as notifications regarding successful or unsuccessful authentication code verifications.

The tracking system 60 may additionally comprise computers at the distributor 74 and/or the provider 80, wherein the distribution authentication module 78 and provider authentication module 84 may alternately be implemented in the computers and the computers are configured to communicate with the server/database 62. In such an embodiment, ACTIVE authentication codes and corresponding additional information may be stored at the computers, and the code reader may query the computer for verification of an authentication code. In another embodiment, the code readers 76, 82 are configured to interface with a communication device configured to communicate with the server/database 62.

In one embodiment, the server/database 62 does not transmit the ACTIVE authentication codes and additional information to the distributor and provider, and the authentication modules at the distributor and provider preferably queries the server/database 62 for verification of an authentication code. It will be appreciated that the system can be implemented in an environment employing a plurality of distributors or intermediate shipping points between the manufacturer and the provider, and that the system described and illustrated is exemplary of one embodiment of the invention.

### Secure Method of Product Distribution Tracking

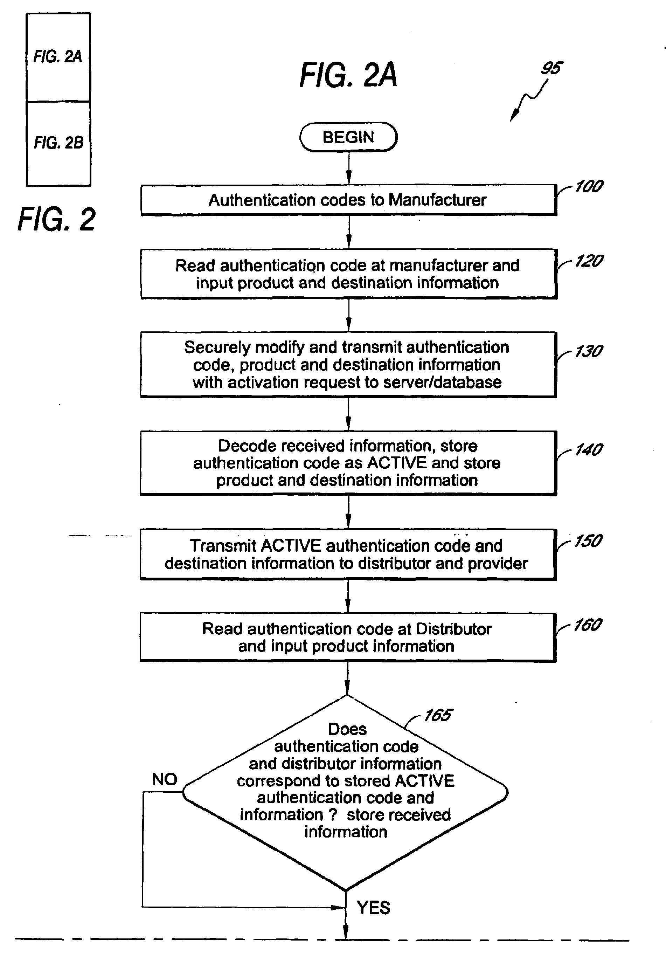

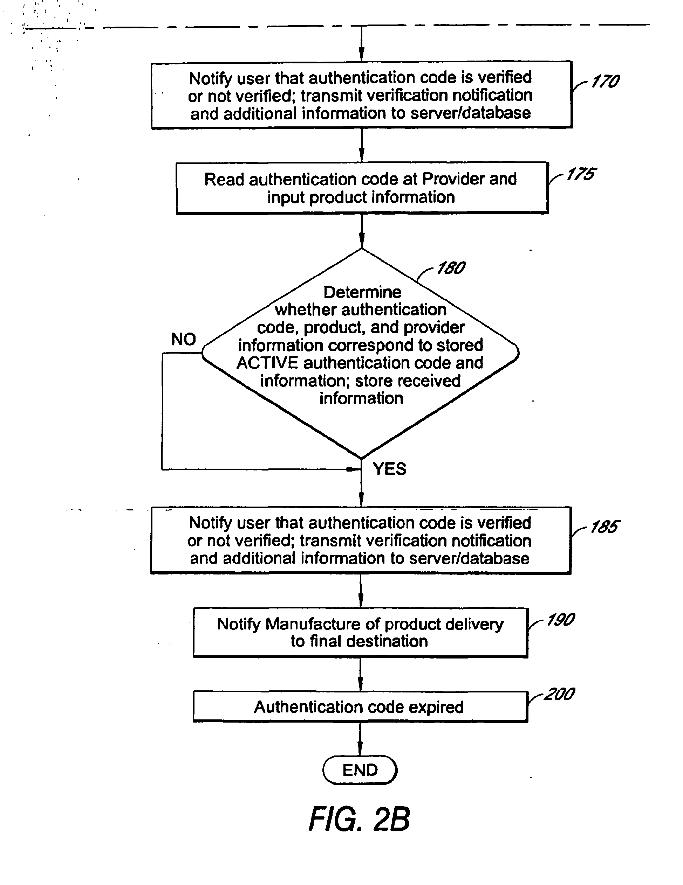

Figures 2A-B are a flow diagram illustrating one embodiment of a method 95 of operating the tracking system 60. In a step 100, the system server/database 62 sends a plurality of authentication codes to the manufacturer 66, and the manufacturer 66 applies or affixes the codes to the product to be shipped or distributed. As previously discussed, the authentication codes may be provided to the manufacturer 66 electronically, such as over the Internet, or manually shipped to the manufacturer. The codes can be affixed by, for example, printing a machine readable version of the codes on a label which is then affixed to the product. Examples of machine readable codes include one dimensional bar codes, two dimensional bar codes, and RFID tags.

In a step 120, the manufacturer inputs an authentication code, affixed to a product, into the computer system 70 by reading the authentication code with the code reader 68. In addition to inputting the authentication code, the manufacturer inputs product and destination information associated with the authentication code, or associates the authentication code with product and destination information already stored at the computer system 70. The destination information may include intermediate destination and final destination information. Thereby, product and destination information are associated with the authentication code within the computer system 70. In a step 130, the code activator module 72 securely modifies or encodes the authentication code and product and destination information, and transmits the encoded information to the server/database 62 along with an activation request. In a step 140, the server/database 62 receives the modified authentication code and corresponding information from the manufacturer 66, and decodes the information. The activation module 65 activates the authentication code by storing the authentication code with an "ACTIVE" status, along with the corresponding additional information or portion thereof from the manufacturer.

In a step 150, the server/database 62 transmits the ACTIVE authentication codes and preferably at least one additional piece of information, such as the destination of the product associated with the ACTIVE authentication code, to the distributor 74 and the provider 80 as designated by the manufacturer 66.

When a product is received at the distributor 74, the distributor 74 inputs the authentication code into the code reader 76 by reading the authentication code on the received product in a step 160. The distributor 74 may also input product information or additional information, such as expected manufacturer information, distributor information, and final destination information. In a step 165, the distribution authentication module 78 determines whether the authentication code corresponds to an ACTIVE authentication code as received from the server/database 62 in step 150. If the distribution authentication module 78 determines that the authentication code corresponds to an ACTIVE authentication code, then the distribution authentication module 78 further determines whether the additional information, such as product, manufacturer, distributor, and/or final destination information correspond to the additional information received from the server/database 62 in connection with the ACTIVE authentication code. Depending on whether the additional information corresponds to that received from the server/database 62, the distribution authentication module 78 stores the received authentication code and additional information as either a verified authentication or failed verification in step 165. In a step 170, the distribution authentication module 78 notifies the distributor of the result of the verification, and transmits the result of the verification and additional information, such as additional distributor information and time of product receipt, to the server/database 62.

The distribution authentication module 78 may be configured to transmit authentication code verification results and corresponding information at predefined times or according to the number of verification attempts, and may be configured to transmit such information once per day, for example, rather than after each verification event. In addition, as will be discussed in further detail hereinafter in connection with Figure 3, the distribution authentication module 78 may query the server/database 62 in the event the authentication code does not correspond to a stored ACTIVE code, and/or the additional information does not correspond to that received from the server/database 62. One embodiment of a method of verification of an authentication code and additional information is discussed in more detail hereinafter below in connection with Figure 3.

In a step 175, the authentication code is read by the code reader 82 at the provider 80, and the provider may also input product or additional information, such as product information and provider information. In a step 180, the provider authentication module 84 determines whether the authentication code read by the code reader 82 corresponds to an ACTIVE authentication code as received from the server/database 62 in step 150. If the authentication code corresponds to an ACTIVE authentication code, then the provider authentication module 84 further determines whether the additional information, such as the provider information, corresponds to the additional information received from the server/database 62. For example, the provider authentication module 84 may determine whether the provider information corresponds to the final destination information provided by the manufacturer 66. Depending on whether the additional information corresponds to that received from the server/database 62, the provider authentication module 84 stores the received authentication code and additional information as either a verified authentication or failed verification in step 180. In a step 185, the provider authentication module 84 notifies the provider of the result of the verification, and transmits the result of the verification and additional information, such as provider information, to the server/database 62.

In a step 190, the system server/database 62 sends a notification to the manufacturer 66 that the product was delivered to the final destination, and any provider information is also transmitted. In a step 200, the authentication code originally activated by the manufacturer 66 and verified by the provider 80 is expired by the system server/database 62 by flagging the authentication code as "EXPIRED". Where a number of distributors or intermediary locations are used along the distribution chain of the product, steps 150 through 170 can be repeated for each distributor or intermediary location to verify the authenticity of the product received and to track the product location.

Excess or surplus authentication codes at the manufacturer create no risk because only authentication codes that have been activated will be verified by the system server/database as corresponding to an authentic product from the manufacturer. In addition, activation of the authentication codes is controlled by the manufacturer, where only the manufacturer receives authentication codes available for activation, and only the manufacturer can activate the codes over a secure communications link using a secure activation scheme.

In one embodiment, manufacturer information, such as the name of the manufacturer and a corresponding identification code, is stored in connection with an ACTIVE authentication code at the system server/database. In this embodiment, a verification request failing to include the expected or stored manufacturer information would result in a failed verification attempt. Thereby, in the event identical authentication codes are inadvertently activated in the tracking system, the server/database will not verify an authentication code unless the entity requesting verification also inputs the expected manufacturer information.

Furthermore, in the event an active authentication code is read more than once at a specified destination, the system can immediately identify the location of the duplicate reading and notify the manufacturer and provider that a product with a duplicate active authentication code has attempted to enter the distribution chain.

In addition, since the location of each authentication code is read and reported to the system server/database along with an authentication code validation request, the product can be tracked from location to location through the distribution chain. Any attempt to read an authentication code read instance which results in an invalid authentication is also recorded with respect to the location where the validation originated. By tracking this information, the point of attempted entry of substitute products can be identified by the system. Furthermore, the number of invalid authentication code readings at a particular location can be tallied and stored in the system server/database such that locations with a large number of invalid readings can be identified as suspect for security problems.

The code activator module 72, authentication module 64 in the system server/database 62, distribution authentication module 78, and provider authentication module 84 are described below in further detail. It will be appreciated that each of these modules can be implemented in a computing system with communications capabilities, or the code activator module 72, distribution authentication module 78, and provider authentication module 84 can be wholly or partially implemented in the code readers 68, 76, 82. Communication with the system server/database 62 preferably uses a secure communications link, such as a secure web link, and a virtual private network can be implemented for the particular members of the distribution chain. The code readers 68, 76, 82 can be implemented with network communication capabilities, such that they can communicate, for example, over the Internet or an intranet with the system server/database 62.

### Authentication Code Verification

Figure 3 is a software flow diagram illustrating one embodiment of a method 300 of processing a verification request at the distribution authentication module 78 or the provider authentication module 84. For simplicity purposes, the description of the method 300 will refer to the distribution authentication module 78. The method 300 begins at a step 301 and proceeds to a step 305. In step 305, the authentication module 78 receives an authentication code and additional information at the code reader 76. The additional information may include, for example, product information, information about the location or entity requesting verification, such as distributor information, final destination information, and product manufacturer information. The additional information received by the authentication module 78 may be retrieved from memory at the code reader 76, such as distributor information. In a step 310, the authentication module 78 determines whether the received authentication code corresponds to an ACTIVE authentication code received from the server/database 62 and stored at the code reader 76. If the authentication module 78 determines in step 310 that yes, the received authentication code corresponds to an ACTIVE authentication code, then the authentication module 78 proceeds to a step 315.

In step 315, the authentication module 78 determines whether the additional information, such as the final destination information, corresponds to the additional information received from the server/database 62 in relation to the ACTIVE authentication code. If the authentication module 78 determines in step 315 that the additional information does correspond to the information received from the server/database 62 in relation to the ACTIVE authentication code, then the authentication module 78 proceeds to a step 320. In step 320, the authentication module 78 stores the additional information or portion thereof in relation to the ACTIVE authentication code as a successful verification, and generates a verification notification to notify the user of the code reader 76 of the successful verification in step 320. The user of the code reader 76 may be notified via an audio or visual indicator, for example. In step 325, the authentication module 78 sends the verification result, authentication code, and additional information to the server/database 62, and the method is terminated in a step 328.

If the authentication module 78 determines in step 310 that the received authentication code does not correspond to an ACTIVE authentication code, then the authentication module 78 proceeds to a step 330. Similarly, if the authentication module 78 determines in step 315 that the additional information does not correspond to the information received from the server/database 62 in relation to the ACTIVE authentication code, then the authentication module 78 proceeds to step 330. In step 330, the authentication module 78 transmits the authentication code, additional information, and a verification request to the server/database 62. The authentication module 64 at the server/database 62 performs steps similar to steps 310 and 315 in response to receipt of the verification request, and generates and sends a response to the verification request according to the determinations regarding whether the authentication code is ACTIVE and whether the additional information corresponds to that stored at the server/database 62. The distribution authentication module 78 receives the response from the server/database 62 regarding the verification request in a step 335, and proceeds to a step 340.

In a step 340, the authentication module 78 determines whether the response to the verification request from the server/database 340 is a notification of a successful or unsuccessful verification of the authentication code sent in step 330. If the authentication module 78 determines in step 340 that the authentication code was verified as ACTIVE and the additional information matched that stored at the server/database 62, then the authentication module 78 proceeds to step 320, which is discussed above. If the authentication module 78 determines in step 340 that the authentication code was not verified as ACTIVE and/or the additional information did not correspond to that stored at the server/database 62, then the authentication module 78 proceeds to a step 345.

In step 345, the authentication code and additional information are stored as a failed verification attempt and the authentication module 78 generates a failed verification notification to notify the user of the code reader 76 of the unsuccessful verification. Notification of the unsuccessful verification may include detailed information as to which information the authentication module 78 or server/database 62 was unable to verify, such as distributor information, manufacturer information, and final destination. Thereby the distributor can identify substitute products or missing products and take the appropriate action. Following step 345 the method 300 proceeds to step 325, discussed above.

In one embodiment, the authentication module 78 is further configured to confirm product type and quantity information, where a combination of codes corresponding to a combination of units such as a mixed shipment, or different codes on different levels of packaging, have been activated by the manufacturer as a single set of authentication codes, and the authentication module 78 can identify any missing codes from the set upon requested authentication of any portion of the set. The authentication module may additionally be configured to notify the manufacturer via the server/database of such findings.

As can be appreciated by one of ordinary skill in the art, each of the modules discussed above comprise various sub-routines, procedures, definitional statements and macros. Each of the modules are typically separately compiled and linked into a single executable program. Therefore, the above description of each of the modules is used for convenience to describe the functionality of the preferred system. Thus, the processes that are undergone by each of the modules may be arbitrarily redistributed to another module in the system.

In addition to authenticity verification upon receipt of the pharmaceutical product from a distributor or manufacturer, point of use validation can also take place where the authentication code on individual dosage medication packaging is read by a code reader for validation at the time the medication is administered or provided to a patient. Thereby, information regarding the date of administration of the medication, information about the patient, and the symptoms exhibited by the patient warranting administration of the medication are communicated to the manufacturer so as to monitor the types of patients, reasons for using the medication, frequency of use of the medication, and time period between manufacture and administration of a medication.

In some embodiments, the code reader at each location in the distribution chain can be preprogrammed with specific information about the location, such as physical location, type of facility, name of facility, and identity of an operator or user. Alternately, the tracking system includes a computing system configured to transmit the specific location information with the authentication code validation request to the system server/database.

For ease and low cost of integration into existing systems, optical scanning codes such as bar codes can be used, and the code reader can be implemented as an optical scanning device such as a bar code scanner, where bar code scanning technology is currently a substantially developed and widely implemented and available technology. Such implementations provide for faster data processing, and therefore faster authentication of the pharmaceutical products.

In certain embodiments, the authentication codes are implemented as the 2-D matrix, or DOT, and the code reader is implemented as an optical scanner configured to read information encoded in the DOT, as described in International Publication No. WO 02/07065. Additional aspects and functions can be added to the secure system implementing the DOT authentication code and optical scanner, such as those described in International Publication No. WO 02/21794.

In one embodiment, the DOT is 7mm in diameter, and comprises 321 white or dark hexagons. The DOT contains 120 bits of Reed-Solomon forward error correction, 32 bits of server routing code, and 128 bits of net data space which provides for 10³⁸ data combinations that can be encrypted. Thus, the DOT can encode a 14 digit GTIN (Global Trade Identification Number) conforming to EAN.UCC format and a 24 digit serial number which can be unique as a fingerprint. The DOT can be printed using current printing technology and conventional inks, print media, and printers, such as thermal and laser printers, and the DOT scanner uses a low cost scan engine and secure link for communication with the system server/database.

The large number of unique data combinations available using the DOT, and the small size of the DOT, make it ideal for use in the pharmaceutical tracking system described herein. In contrast to bar codes, the DOT can be applied to individual dosage packaging without the use of excess packaging to provide enough space for the code. Because of the extensive number of data combinations and corresponding codes that can be encrypted in a single DOT, a different DOT can be applied to each unit at the smallest packaging level without running out of unique data combinations or codes. Thereby, the smallest packaging level of a pharmaceutical product, such as individual or single dose packaging, can be authenticated and tracked from the point of manufacture to actual administration or use of the product.

## Claims

1. A pharmaceutical product distribution system (60), comprising:
an activation computer (70) configured to store authentication codes in a database (62) and associate said authentication codes with pharmaceutical packages, wherein said activation computer (70) is further configured to activate said authentication codes for pharmaceutical packages that are being sent to destination sites; and
an authentication computer configured to receive authentication codes that are read from pharmaceutical packages received at said destination sites and determine whether said authentication codes have been activated.

2. The system of Claim 1, wherein said activation computer (70) is configured to store additional information for said pharmaceutical packages in said database (62) along with said activated authentication code.

3. The system of Claim 2, wherein the authentication computer is configured to determine whether information received in connection with an authentication code corresponds to the additional information stored at said database (62).

4. The system of Claim 2, wherein said additional information includes at least one of: pharmaceutical package destination information, pharmaceutical type, pharmaceutical quantity, pharmaceutical dosage, or pharmaceutical manufacturer information.

5. The system of Claim 1, wherein the authentication code is a machine readable code.

6. The system of Claim 5, wherein said authentication computer is configured to receive authentication codes from a code reader (82).

7. The system of Claim 1, further comprising a server (62) in communication with said database (62), said activation computer (70), and said authentication computer, wherein said server (62) transmits an activated authentication code to said authentication computer, and said authentication computer is configured to store the activated authentication code received from said server.

8. The system of Claim 2, wherein said additional information comprises the intended destination of said pharmaceutical packages.

9. The system of Claim 8, wherein the intended destination includes intermediate and final destination information.

10. The system of Claim 1, wherein said authentication computer is configured to notify said activation computer (70) in response to determining that a received authentication code corresponding to a pharmaceutical package is not an activated authentication code.

11. The system of Claim 1 further comprising :
a system server (62) comprising said activation computer (70) and including a first authentication module (64);
a first code reader (68) configured to read authentication codes from product packaging; and
an activation module (65) configured to receive an authentication code read by said first code reader (68) from product packaging prior to distribution, and to transmit an activation request to the system server (62), wherein said activation request comprises the authentication code read by said first code reader (68), and wherein the system server (62) stores the authentication code as an active authentication code in response to the activation request.

12. The system of Claim 11, further comprising at least a second code reader (76) configured to read authentication codes from product packaging, and a second authentication module (78), configured to receive an authentication code read by said second code reader (76) from product packaging and active authentication codes from said system server (62), and configured to determine whether said authentication code read by said second code reader (76) corresponds to said active authentication code received from said system server (62), and to notify a user of said second code reader (76) as to whether the authentication code read by said second code reader (76) corresponds to said active authentication code.

13. The system of Claim 12, wherein said second authentication module (78) is further configured to transmit a verification request and said authentication code read by said second code reader (78) to said system server (62), wherein said first authentication module (64) is further configured to determine whether said authentication code received with said authentication request corresponds to an active authentication code stored at said system server (62), and wherein the first authentication module (64) is configured to notify said second authentication module (78) as to whether said authentication code received with said verification request corresponds to an active authentication code.

14. The system of Claim 11, wherein said activation request includes destination information for the product, said destination information is stored at the system server (62) in connection with the active authentication code, said second authentication module (78) Is configured to receive said destination information from said system server (62) with said active authentication code, and said second authentication module (78) is further configured to determine whether information at said second code reader (76) corresponds to said destination information received from said system server (62).

15. The system of Claim 14, wherein said destination information includes intermediate destination information and final destination information.

16. The system of Claim 12, wherein said first authentication module (64) is configured to store said previously active authentication code as an expired authentication code when said second authentication module (78) determines that said authentication module read by said second code reader (76) corresponds to said active authentication module.

17. The system of Claim 11, wherein said activation module (65) is further configured to encode said activation request, and to transmit said encoded activation module to said system server (62), and wherein said system server (62) is configured to decode said encoded activation request.

18. A method (95) of tracking and authenticating pharmaceutical products from an origin location to a final destination, comprising:
applying (100) an authentication code to product packaging;
reading (120) the authentication code from the product packaging at the origin location prior to distribution;
sending (130) an activation request to a system server (62) wherein said activation request includes the read authentication code; and
activating (140) the read authentication code in response to the activation request, comprising storing the read authentication code as an active authentication code at the system server (62).

19. The method of Claim 18, further comprising:
sending (150) the active authentication code to a product receiving location (74, 80);
reading (160, 175) an authentication code from product packaging received at a receiving location (74, 80);
determining (165, 180) whether said authentication code read at said receiving location (74, 80) corresponds to said active authentication code; and
notifying (170, 185) a user of the result of said determination.

20. The method of Claim 19, further comprising sending (165, 180) a verification request and said authentication code read at said receiving location (74, 80) to said system server (62), determining whether said authentication code read at said receiving location (74, 80) corresponds to an active authentication code stored at said system server (62), and notifying (170, 185) the receiving location (74, 80) as to whether the authentication code read from product packaging at the receiving location (74, 80) corresponds to an active authentication code stored at the system server (62).

21. The method of Claim 19, further comprising expiring (200) the active authentication code in response to determining that the authentication code read at the receiving location (74, 80) corresponds to the active authentication code, wherein expiring comprises storing the active authentication code at the system server (62) as an expired authentication code.

22. The method of Claim 18, wherein said activation request includes destination information for the product, and wherein activating further comprises storing said destination information in connection with said active authentication code.

23. The method of Claim 18, wherein said activation request includes destination information for the product, wherein activating further comprises storing said destination information in connection with said active authentication code at said system server (62), and wherein the method further comprises sending said destination information to a receiving location (74, 80), determining whether receiving location information corresponds to said destination information from said system server (62), and notifying the receiving location (74, 80) as to whether the receiving location information corresponds to said destination information.

24. The method of Claim 18, further comprising encoding said activation request, sending said encoded activation request to said system server (62), and decoding said encoded activation request at said server (62) prior to said activating.

## Patentansprüche

1. System zum Vertrieb von pharmazeutischen Produkten (60), umfassend:
einen Aktivierungscomputer (70), der konfiguriert ist, um Authentifizierungscodes in einer Datenbank (62) zu speichern, und die Authentifizierungscodes mit pharmazeutischen Packungen zu assoziieren, wobei der Aktivierungscomputer (70) weiter konfiguriert ist, um die Authentifizierungscodes für pharmazeutische Packungen zu aktivieren, die an Ziel-Positionen geschickt werden; und
einen Authentifizierungscomputer, der konfiguriert ist, um Authentifizierungscodes zu empfangen, die von pharmazeutischen Packungen gelesen werden, die an den Ziel-Positionen empfangen werden, und zu bestimmen, ob die Authentifizierungscodes aktiviert wurden.

2. System nach Anspruch 1, wobei der Aktivierungscomputer (70) konfiguriert ist, um zusätzliche Information für die pharmazeutischen Packungen in der Datenbank (62) zu speichern, zusammen mit dem aktivierten Authentifizierungscode.

3. System nach Anspruch 2, wobei der Authentifizierungscomputer konfiguriert ist, um zu bestimmen, ob die Information, die in Verbindung mit einem Authentifizierungscode empfangen wurde, der zusätzlichen Information entspricht, die in der Datenbank (62) gespeichert ist.

4. System nach Anspruch 2, wobei die zusätzliche Information mindestens eines von Information über das Ziel der pharmazeutischen Packung, die pharmazeutische Art, die pharmazeutische Menge, die pharmazeutische Dosierung oder den pharmazeutischen Hersteller umfasst.

5. System nach Anspruch 1, wobei der Authentifizierungscode ein maschinenlesbarer Code ist.

6. System nach Anspruch 5, wobei der Authentifizierungscomputer konfiguriert ist, um Authentifizierungscodes von einem Codeleser (82) zu empfangen.

7. System nach Anspruch 1, weiter umfassend einen Server (62) in Kommunikation mit der Datenbank (62), dem Aktivierungscomputer (70) und dem Authentifizierungscomputer, wobei der Server (62) einen aktivierten Authentifizierungscode an den Authentifizierungscomputer überträgt, und der Authentifizierungscomputer konfiguriert ist, um den aktivierten Authentifizierungscode, empfangen von dem Server, zu speichern.

8. System nach Anspruch 2, wobei die zusätzliche Information das beabsichtigte Ziel der pharmazeutischen Packungen umfasst.

9. System nach Anspruch 8, wobei das beabsichtigte Ziel Information über das Zwischen- und das Endziel umfasst.

10. System nach Anspruch 1, wobei der Authentifizierungscomputer konfiguriert ist, um den Aktivierungscomputer (70) als Antwort auf die Bestimmung zu benachrichtigen, dass ein empfangener Authentifizierungscode, der einer pharmazeutischen Packung entspricht, kein aktivierter Authentifizierungscode ist.

11. System nach Anspruch 1, weiter umfassend
einen Systemserver (62), umfassend den Aktiviertutgscomputer (70) und enthaltend ein erstens Authentifizierungsmodul (64);
einen ersten Codeleser (68), der konfiguriert ist, um Authentifizierungscodes von der Produktpackung zu lesen; und
ein Aktivierungsmodul (65), das konfiguriert ist, um einen Authentifizierungscode zu empfangen, der vom ersten Codeleser (68) von der Produktpackung vor dem Vertrieb gelesen wurde, und um eine Aktivierungsanfrage an den Systemserver (62) zu übertragen, wobei die Aktivierungsanfrage den Authentifizierungscode umfasst, der vom ersten Codeleser (68) gelesen wurde, und wobei der Systemserver (62) den Authentifizierungscode als einen aktiven Authentifizierungscode als Antwort auf die Aktivierungsanfrage speichert.

12. System nach Anspruch 11, weiter umfassend mindestens einen zweiten Codeleser (76), der konfiguriert ist, um Authentifizierungscodes von der Produktpackung zu lesen, und ein zweites Authentifizierungsmodul (78), das konfiguriert ist, um einen Authentifizierungscode zu empfangen, der vom zweiten Codeleser (76) von der Produktpackung gelesen wurde, und aktive Authentifizierungscodes vom Systemserver (62), und konfiguriert, um zu bestimmen, ob der Authentifizierungscode, der von dem zweiten Codeleser (76) gelesen wurde, dem aktiven Authentifizierungscode entspricht, der von dem Systemserver (62) empfangen wurde, und einem Benutzer des zweiten Codelesers (76) mitzuteilen, ob der Authentifizierungscode, der von dem zweiten Codeleser (76) gelesen wurde, dem aktiven Authentifizierungscode entspricht.

13. System nach Anspruch 12, wobei das zweite Authentifizierungsmodul (78) weiter konfiguriert ist, um eine Überprüffingsanfrage und den Authentifizierungscode, der von dem zweiten Codeleser (78) gelesen wurde, an den Systemserver (62) zu übertragen, wobei das erste Autbentifizierungsmodul (64) weiter konfiguriert ist, um zu bestimmen, ob der Authentifizierungscode, der mit der Authentifizierungsanftage empfangen wurde, dem aktiven Authentifizierungscrode entspricht, der in dem Systemserver (62) gespeichert ist, und wobei das erste Authentifizierungsmodul (64) konfigurient ist, und dem zweiten Authentifizierungsmodul (78) mitzuteilen, ob der Authentifizierungscode, der mit der Überprüfungsanfrage empfangen wurde, einem aktiven Authentifizierungscode entspricht.

14. System nach Anspruch 11, wobei die Aktivierungsanfrage die Zielinformation für das Produkt umfasst, wobei die Zielinformation im Systemserver (62) in Verbindung mit dem aktiven Authentifizierungscode gespeichert ist, wobei das zweite Authentifizierungsmodul (78) konfiguriert ist, um die Zielinformation von dem Systemserver (62) mit dem aktiven Authentifizierungscode zu empfangen, und das zweite Authentifizierungsmodul (78) weiter konfiguriert ist, um zu bestimmen, ob die Information in dem zweiten Codeleser (76) der Zielinformation entspricht, die vom dem Systemserver (62) erhalten wurde.

15. System nach Anspruch 14, wobei die Zielinformation Information über das Zwischenziel und Information über das Endziel umfasst.

16. System nach Anspruch 12, wobei das erste Authentifizierungsmodul (64) konfiguriert ist, um den vorher aktiven Authentifizierungscode als einen abgelaufenen Authentifizierungscode zu speichern, wenn das zweite Authentifizierungsmodul (78) bestimmt, dass das Autlientifizierungsmodul, das vom zweiten Codeleser (76) gelesen wurde, dem aktiven Authentifizierungsmodul entspricht.

17. System nach Anspruch 11, wobei das Aktivierungsmodul (65) weiter konfiguriert ist, um die Aktivierungsanfrage zu codieren und das codierte Aktivierungsmodul an den Systemserver (62) zu übertragen, und wobei der Systemserver (62) konfiguriert ist, um die codierte Aktivierungsanfrage zu decodieren.

18. Verfahren (95) zur Nachverfolgung und Authentifizierung von pharmazeutischen Produkten von einer Ursprungsposition bis zu einem Endziel, umfassend:
Aufbringung (100) eines Authentifizierungscodes auf die Produktpackung;
Lesen (120) des Authentifizierungscodes von der Produktpackung an der Ursprungsposition vor dem Vertrieb;
Sendung (130) einer Aktivierungsanfrage an einen Systemserver (62), wobei die Aktivierungsanfrage den gelesenen Authentifizierungscode umfasst; und
Aktivierung (140) des gelesenen Authentifizierungscodes als Antwort auf die Aktivierungsanfrage, umfassend die Speicherung des gelesenen Authentifizierungscodes als einen aktiven Authentifizierungscode im Systemserver (62).

19. Verfahren nach Anspruch 18, weiter umfassend:
Sendung (150) des aktiven Authentifizierungscodes an eine Produktempfangsposition (74, 80);
Lesen (160, 175) eines Authentifizierungscodes von der Produktpackung, die an einer Empfangsposition (74, 80) empfangen wurde;
Bestimmung (165, 180), ob der Authentifizierungscode, der an der Empfangsposition (74, 80) gelesen wurde, dem aktiven Authentifizierungscode entspricht; und
Benachrichtigung (170, 185) eines Benutzers vom Ergebnis der Bestimmung.

20. Verfahren nach Anspruch 19, weiter umfassend Sendung (165, 180) einer Überprüfungsanfrage und des Authentifizierungscodes, der an der Empfangsposition (74, 80) gelesen wurde, an den Systemserver (62), Bestimmung, ob der Authentifizierungscode, der an der Empfangsposition (74, 80) gelesen wurde, einem aktiven Authentifizierungscode entspricht, der im Systemserver (62) gespeichert ist, und Benachrichtigung (170, 185) der Empfangsposition (74, 80), ob der Authentifizierungscode, der von der Produktpackung an der Empfangsposition (74, 80) gelesen wurde, einem aktiven Authentifizierungscode entspricht, der im Systemserver (62) gespeichert ist.

21. Verfahren nach Anspruch 19, weiter umfassend Ablaufenlassen (200) des aktiven Authentifizierungscodes als Antwort auf die Bestimmung, dass der Authentifizierungscode, der an der Empfangsposition (74, 80) gelesen wurde, dem aktiven Authentifizierungscode entspricht; wobei das Ablaufenlassen die Speicherung des aktiven Authentifizieningscodes im Systemserver (62) als einen abgelaufenen Authentifizierungscode umfasst.

22. Verfahren nach Anspruch 18, wobei die Aktivierungsanfrage Zielinformation für das Produkt umfasst, und wobei die Aktivierung weiter die Speicherung der Zielinformation in Verbindung mit dem aktiven Authentifizierungscode umfasst.

23. Verfahren nach Anspruch 18, wobei die Aktivierungsanfrage Zielinformation für das Produkt umfasst, und wobei die Aktivierung weiter die Speicherung der Zielinformation in Verbindung mit dem aktiven Authentifizierungscode in dem Systemserver (62) umfasst, und wobei das Verfahren weiter die Sendung der Zielinformation an eine Empfangsposition (74, 80) umfasst, die Bestimmung, ob die Empfangspositionsinformation der Zielinformation von dem Systemserver (62) entspricht, und Benachrichtigung der Empfangsposition (74, 80), ob die Empfangspositionsinformation der Zielinformation entspricht.

24. Verfahren nach Anspruch 18, weiter umfassend Codierung der Aktivienangsanfrage, Sendung der codierten Aktivierungsanfrage an den Systemserver (62), und Decodierung der codierten Aktivierungsanfrage an dem Server (62) vor der Aktivierung.

## Revendications

1. Système de distribution de produits pharmaceutiques (60), comprenant :
un ordinateur d'activation (70) configuré de manière à stocker des codes d'authentification dans une base de données (62) et à associer lesdits codes d'authentification à des conditionnements pharmaceutiques, dans lequel ledit ordinateur d'activation (70) est en outre configuré de manière à activer lesdits codes d'authentification pour des conditionnements pharmaceutiques qui sont envoyés à des sites de destination; et
un ordinateur d'authentification configuré de manière à recevoir des codes d'authentification qui sont lus à partir de conditionnements pharmaceutiques reçus au niveau desdits sites de destination, et à déterminer si lesdits codes d'authentification ont été activés.

2. Système selon la revendication 1, dans lequel ledit ordinateur d'activation (70) est configuré de manière à stocker des informations supplémentaires pour lesdits conditionnements pharmaceutiques dans ladite base de données (62) avec ledit code d'authentification activé.

3. Système selon la revendication 2, dans lequel l'ordinateur d'authentification est configuré de manière à déterminer si des informations reçues en association avec un code d'authentification correspondent aux informations supplémentaires stockées dans ladite base de données (62).

4. Système selon la revendication 2, dans lequel lesdites informations supplémentaires incluent au moins un élément parmi : les informations de destination de conditionnements pharmaceutiques, le type pharmaceutique, la quantité pharmaceutique, le dosage pharmaceutique, ou les informations connexes à des fabricants de produits pharmaceutiques.

5. Système selon la revendication 1, dans lequel le code d'authentification est un code lisible par machine.

6. Système selon la revendication 5, dans lequel ledit ordinateur d'authentification est configuré de manière à recevoir des codes d'authentification en provenance d'un lecteur de codes (82).

7. Système selon la revendication 1, comprenant en outre un serveur (62) en communication avec ladite base de données (62), ledit ordinateur d'activation (70), et ledit ordinateur d'authentification, dans lequel ledit serveur (62) transmet un code d'authentification activé audit ordinateur d'authentification, et ledit ordinateur d'authentification est configuré de manière à stocker le code d'authentification activé reçu à partir dudit serveur.

8. Système selon la revendication 2, dans lequel lesdites informations supplémentaires comprennent la destination attendue desdits conditionnements pharmaceutiques.

9. Système selon la revendication 8, dans lequel la destination attendue inclut des informations de destination intermédiaire et de destination finale.

10. Système selon la revendication 1, dans lequel ledit ordinateur d'authentification est configuré de manière à avertir ledit ordinateur d'activation (70) en réponse à la détermination qu'un code d'authentification reçu correspondant à un conditionnement pharmaceutique n'est pas un code d'authentification activé.

11. Système selon la revendication 1, comprenant en outre :
un serveur de système (62) comprenant ledit ordinateur d'activation (70) et incluant un premier module d'authentification (64) ;
un premier lecteur de codes (68) configuré de manière à lire des codes d'authentification à partir d'un conditionnement de produit ; et
un module d'activation (65) configuré de manière à recevoir un code d'authentification lu par ledit premier lecteur de codes (68) à partir d'un conditionnement de produit préalablement à une distribution, et à transmettre une demande d'activation au serveur de système (62), dans lequel ladite demande d'activation comprend le code d'authentification lu par ledit premier lecteur de codes (68), et dans lequel le serveur de système (62) stocke le code d'authentification sous la forme d'un code d'authentification actif, en réponse à la demande d'activation.

12. Système selon la revendication 11, comprenant en outre au moins un second lecteur de codes (76) configuré de manière à lire des codes d'authentification à partir d'un conditionnement de produit, et un second module d'authentification (78) configuré de manière à recevoir un code d'authentification lu par ledit second lecteur de codes (76) à partir d'un conditionnement de produit et des codes d'authentification actifs à partir dudit serveur de système (62), et configuré de manière à déterminer si ledit code d'authentification lu par ledit second lecteur de codes (76) correspond audit code d'authentification actif reçu à partir dudit serveur de système (62), et à signaler à un utilisateur dudit second lecteur de codes (76) si le code d'authentification lu par ledit second lecteur de codes (76) correspond ou non audit code d'authentification actif.

13. Système selon la revendication 12, dans lequel ledit second module d'authentification (78) est en outre configuré de manière à transmettre une demande de vérification et ledit code d'authentification lu par ledit second lecteur de codes (78) audit serveur de système (62), dans lequel ledit premier module d'authentification (64) est en outre configuré de manière à déterminer si ledit code d'authentification reçu avec ladite demande d'authentification correspond au code d'authentification actif stocké au niveau dudit serveur de système (62), et dans lequel le premier module d'authentification (64) est configuré de manière à signaler audit second module d'authentification (78) si ledit code d'authentification reçu avec ladite demande de vérification correspond à un code d'authentification actif.

14. Système selon la revendication 11, dans lequel ladite demande d'activation inclut des informations de destination du produit, lesdites informations de destination sont stockées au niveau du serveur de système (62) conjointement au code d'authentification actif, ledit second module d'authentification (78) est configuré de manière à recevoir lesdites informations de destination à partir dudit serveur de système (62) avec ledit code d'authentification actif, et ledit second module d'authentification (78) est en outre configuré de manière à déterminer si des informations au niveau dudit second lecteur de codes (76) correspondent auxdites informations de destination reçues à partir dudit serveur de système (62).

15. Système selon la revendication 14, dans lequel lesdites informations de destination comprennent des informations de destination intermédiaire et des informations de destination finale.

16. Système selon la revendication 12, dans lequel ledit premier module d'authentification (64) est configuré de manière à stocker ledit code d'authentification précédemment actif sous la forme d'un code d'authentification échu lorsque ledit second module d'authentification (78) détermine que ledit module d'authentification lu par ledit second lecteur de codes (76) correspond audit module d'authentification actif.

17. Système selon la revendication 11, dans lequel ledit module d'activation (65) est en outre configuré de manière à coder ladite demande d'activation, et à transmettre ledit module d'activation codé audit serveur de système (62), et dans lequel ledit serveur de système (62) est configuré de manière à décoder ladite demande d'activation codée.

18. Procédé (95) de suivi et d'authentification de produits pharmaceutiques depuis un emplacement d'origine jusqu'à une destination finale, comprenant :
l'application (100) d'un code d'authentification à un conditionnement de produit ;
la lecture (120) du code d'authentification à partir du conditionnement de produit au niveau de l'emplacement d'origine, préalablement à une distribution ;
l'envoi (130) d'une demande d'activation à un serveur de système (62), dans lequel ladite demande d'activation inclut le code d'authentification lu ; et
l'activation (140) du code d'authentification lu en réponse à la demande d'activation, comprenant le stockage du code d'authentification lu, sous la forme d'un code d'authentification actif, au niveau dudit serveur de système (62).

19. Procédé selon la revendication 18, comprenant en outre :
l'envoi (150) du code d'authentification actif à un site de réception de produits (74, 80) ;
la lecture (160, 175) d'un code d'authentification à partir d'un conditionnement de produit reçu au niveau d'un site de réception (74, 80) ;
la détermination (165, 180) du fait si ledit code d'authentification lu au niveau dudit site de réception (74, 80) correspond ou non audit code d'authentification actif ; et
le signalement (170, 185), à un utilisateur, du résultat de ladite détermination.

20. Procédé selon la revendication 19, comprenant en outre l'envoi (165, 180) d'une demande de vérification et dudit code d'authentification lu au niveau dudit site de réception (74, 80) audit serveur de système (62), la détermination du fait si ledit code d'authentification lu au niveau dudit site de réception (74, 80) correspond à un code d'authentification actif stocké au niveau dudit serveur de système (62), et le signalement (170, 185), au site de réception (74, 80), du fait si le code d'authentification lu à partir du conditionnement de produit au niveau du site de réception (74, 80) correspond ou non à un code d'authentification actif stocké au niveau du serveur de système (62).

21. Procédé selon la revendication 19, comprenant en outre la déclaration d'expiration (200) du code d'authentification actif en réponse à la détermination que le code d'authentification lu au niveau du site de réception (74, 80) correspond au code d'authentification actif, dans lequel l'expiration comprend le stockage du code d'authentification actif au niveau du serveur de système (62), sous la forme d'un code d'authentification échu.

22. Procédé selon la revendication 18, dans lequel ladite demande d'activation comprend des informations de destination du produit, et dans lequel l'activation comprend en outre le stockage desdites informations de destination conjointement audit code d'authentification actif.

23. Procédé selon la revendication 18, dans lequel ladite demande d'activation comprend des informations de destination du produit, dans lequel l'activation comprend en outre le stockage desdites informations de destination conjointement audit code d'authentification actif au niveau dudit serveur de système (62), et dans lequel le procédé comprend en outre l'envoi desdites informations de destination à un site de réception (74, 80), la détermination du fait si les informations de site de réception correspondent auxdites informations de destination en provenance dudit serveur de système (62), et le signalement au site de réception (74, 80) du fait si les informations de site de réception correspondent ou non auxdites informations de destination.

24. Procédé selon la revendication 18, comprenant en outre le codage de ladite demande d'activation, l'envoi de ladite demande d'activation codée audit serveur de système (62), et le décodage de ladite demande d'activation codée au niveau dudit serveur (62) préalablement à ladite activation.
